# EUROPÄISCHE PATENTANMELDUNG

(11) **EP 4 668 286 A1**
(43) Veröffentlichungstag der Anmeldung: **24.12.2025**
(21) Anmeldenummer: 24182489.5
(22) Anmeldetag: 17.06.2024
(51) Int. Cl.: G16H 40/40, G16H 40/60

(54) **SCHNITTSTELLENVORRICHTUNG FÜR MEDIZINTECHNISCHE GERÄTE UND VERFAHREN**

(71) Anmelder: Fresenius Medical Care Deutschland GmbH, 61352 Bad Homburg (DE)
(72) Erfinder: WEGNER, Stefan, 61352 Bad Homburg (DE)
(74) Vertreter: Hitzelberger, Christoph Michael

(57) **Zusammenfassung**

Die vorliegende Erfindung offenbart eine Schnittstellenvorrichtung zum Datenaustausch von medizintechnischen Geräten mit entfernten Vorrichtungen über Netzwerke und entsprechenden Verfahren hierzu, wobei sichergestellt wird, dass der Datentransfer von der medizinischen Vorrichtung zur Schnittstellenvorrichtung zuverlässig auslesbar ist und der Datenaustausch mit der zumindest einen entfernten Vorrichtungen robust gegenüber unerwünschtem Datenaustausch ist.

## Beschreibung

### Technisches Gebiet

Die vorliegende Erfindung liegt auf dem Gebiet der Medizintechnik, insbesondere auf dem Gebiet von Schnittstellenvorrichtungen zum Datenaustausch von medizintechnischen Geräten mit entfernten Vorrichtungen über Netzwerke und entsprechenden Verfahren hierzu.

### Hintergrund

Bei der Verwendung medizintechnischer Geräte, insbesondere solchen, welche direkt zur Behandlung von Patienten Verwendung finden, fallen umfangreiche Daten an. Diese Daten umfassen Daten, die sich auf Betriebszustände des medizintechnischen Geräts oder damit verbundenen weiteren Vorrichtungen beziehen, als auch Daten, die sich auf einen mit dem medizintechnischen Gerät behandelten Patienten beziehen. Derartige Daten werden zur Überwachung, Steuerung und/oder Regelung des medizintechnischen Geräts oder einer damit ausgeführten medizinischen Behandlung verwendet.

Die Datenintegrität und Sicherheit ist für derartige Daten dementsprechend von besonderer Bedeutung, da von dieser die Sicherheit und der Behandlungserfolg des Patienten abhängt.

In der Vergangenheit war die Verwendung derartiger Daten auf das medizintechnische Gerät selbst beschränkt und diese wurden beispielsweise zur Darstellung von Betriebszuständen oder Behandlungszuständen auf Anzeigemitteln wie beispielsweise einem Bildschirm verwendet. Derartige medizintechnische Geräte verfügen oftmals lediglich über eingeschränkte Datenaustauschmöglichkeiten wie serielle Datenschnittstellen, beispielsweise RS232 Schnittstellen, die nicht zum Datenaustausch mit modernen Netzwerkstandards zum Datenaustausch mit beispielsweise Krankenhausnetzwerken oder dem Internet eingerichtet sind.

Im Zuge der Verbreitung von netzwerkgestützten Systemen zur Überwachung, Dokumentation, Regelung und/oder Steuerung von medizintechnischen Geräten und damit ausgeführten medizinischen Behandlungen besteht der Bedarf, eine Möglichkeit zu schaffen, medizintechnische Geräte in moderne Netzwerke einzubinden und hierbei die Sicherheit und Datenintegrität der Daten des medizintechnischen Geräts zu gewährleisten und insbesondere vor Angriffen aus diesen Netzwerken, wie beispielsweise unter dem Oberbegriff Cyberattacken bekannt, zu schützen.

### Zusammenfassung der Erfindung

Die vorliegende Erfindung hat demnach die Aufgabe, eine Möglichkeit zu schaffen, Daten von einem medizintechnischen Gerät aufzunehmen und zumindest einer entfernten Vorrichtung über zumindest ein Netzwerk zugänglich zu machen, wobei sichergestellt wird, dass die Daten des medizintechnischen Geräts zuverlässig auslesbar sind und der Datenaustausch mit der zumindest einen entfernten Vorrichtung robust gegenüber unerwünschtem Datenaustausch ist.

Gelöst wird diese Aufgabe durch eine Schnittstellenvorrichtung nach Anspruch 1, ein medizintechnisches Gerät nach Anspruch 11, ein Verfahren nach Anspruch 13, ein Softwareprodukt nach Anspruch 14 und ein maschinenlesbares Speichermedium nach Anspruch 15.

Die Unteransprüche 2 bis 10 und 12 beschreiben vorteilhafte Ausführungsformen.

### Kurzbeschreibung der Zeichnungen

Weitere Einzelheiten und Vorteile der Erfindung werden anhand von in den Zeichnungen dargestellten Ausführungsbeispielen näher beschrieben.

Es zeigen
die Figur 1 eine beispielhafte schematische Darstellung einer erfindungsgemäßen Schnittstelle 100 und eine typische Verwendung der erfindungsgemäßen Schnittstelle 100, und
die Figur 2 eine weitere schematische Darstellung einer erfindungsgemäßen Schnittstelle 100.

### Detaillierte Beschreibung

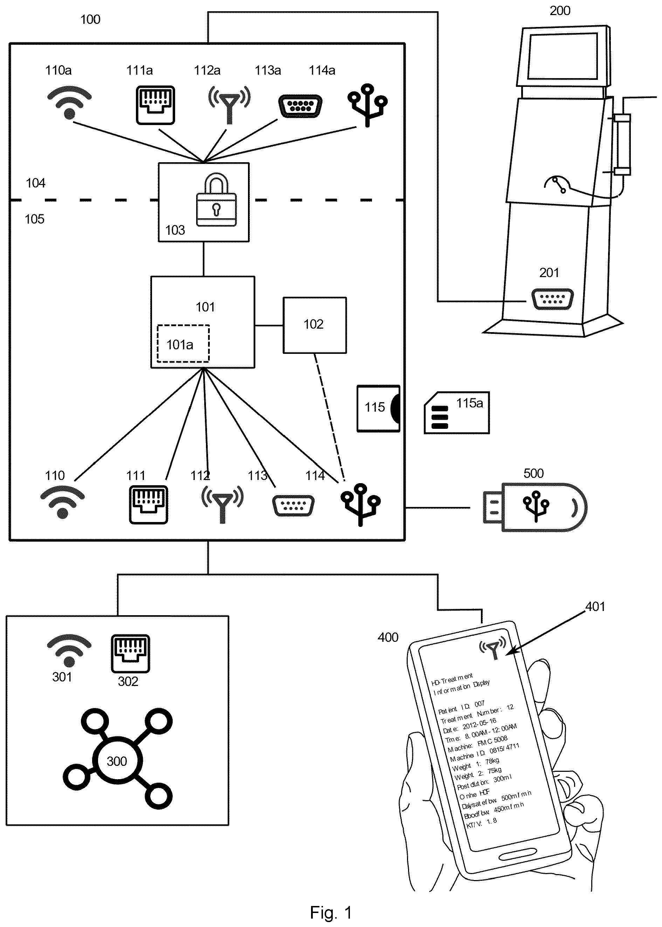

Die Schnittstellenvorrichtung 100, welche in Figur 1 dargestellt ist, umfasst einen integrierten Schaltkreis 101, welcher beispielsweise als CPU oder Mikrokontroller ausgeführt sein kann. Der integrierte Schaltkreis wird durch eine Software programmiert und hierdurch zur Steuerung der Schnittstellenvorrichtung 100 konfiguriert. Die Software, welche hierzu verwendet wird, wird in der Speichervorrichtung 102 gespeichert. Die Speichervorrichtung 102 kann beispielsweise als EPROM, EEPROM, Flash Memory Speicher oder als sonstiger elektronischer Speicher ausgeführt sein, welcher wiederbeschreibbar ist und dessen Speicherinhalt nichtflüchtig erhalten bleibt.

Die Schnittstellenvorrichtung 100 umfasst eine Vielzahl von Schnittstellen, welche zum Datenaustausch mit zumindest einer entfernten Vorrichtung über zumindest ein Netzwerk eingerichtet sind. Diese Schnittstellen sind in Figur 1 beispielhaft als WLAN-Schnittstelle 110, LAN-Schnittstelle 111, Mobilfunk-Schnittstelle 112, serielle Schnittstelle 113, beispielhaft als RS232 Schnittstelle ausgeführt und eine weitere serielle Schnittstelle 114, beispielhaft als USB-Schnittstelle ausgeführt. Ein weitere in Figur 1 nicht dargestellte Schnittstelle ist die I2C (englisch I-square-C) Schnittstelle. Ebenfalls kann auch eine Schnittstelle zur Aufnahme von Speicherkarten, wie der SD-Slot 115 zur Aufnahme einer SD-Speicherkarte 115a, vorhanden sein. Diese kann insbesondere auch zur Aktualisierung der Software, die in der Speichervorrichtung 102 gespeichert ist, verwendet werden, in dem die aktualisierte Software auf einer Speicherkarte vorliegt.

Entfernte Vorrichtungen können beispielsweise Computer sein, welche über ein klinisches Netzwerk Daten austauschen, mobile Computer, wie Laptops oder Tabletcomputer, welche beispielsweise einem Techniker zugeordnet sind und über WLAN, USB, Bluetooth oder RFID Daten austauschen, oder Mobilfunkgeräte wie Smartphones, die typischerweise über WLAN oder Mobilfunk kommunizieren.

In der Figur 1 ist beispielhaft ein klinisches Netzwerk 300 symbolisiert, welches Daten über WLAN 301 oder LAN 302 austauscht. Weiterhin ist in der Figur 1 ein mobiles Endgerät, welches als Smartphone 400 ausgeführt ist, dargestellt und welches Daten über eine Mobilfunkverbindung 401 austauscht.

Weiterhin zeigt die Figur 1 eine USB-Speichervorrichtung 500, welche über eine entsprechende Schnittstelle 114 Daten mit der Schnittstellenvorrichtung 100 austauschen kann. Dies ist in Figur 1 durch die gestrichelte Linie von der USB - Schnittstelle 114 zur Speichervorrichtung 102 symbolisiert. Auf diese Weise kann ebenfalls die Software, welche den integrierten Schaltkreis 101 steuert, in die Speichervorrichtung 102 eingespeichert werden. Es ist aber auch möglich, die Software über eine der anderen Schnittstellen 110 bis 113 und 115 auf die Speichervorrichtung 102 aufzuspeichern.

Die Schnittstellenvorrichtung 100 verfügt weiterhin über zumindest eine Schnittstelle 110a, 111a, 112a, 113a und/oder 114a, welche korrespondierend zu den Schnittstellen 110 bis 114 ausgeführt sind. Grundsätzlich kann die Schnittstellenvorrichtung jedwede geeignete Datenschnittstelle umfassen, die dazu eingerichtet ist, Daten mit einer entfernten Vorrichtung 300, 400 und/oder einem medizintechnischen Gerät 200 auszutauschen oder von dort zu empfangen. Derartige Schnittstellen sind nicht auf serielle Schnittstellen, parallele Schnittstellen, RS232-Schnittstellen, 12C-Schnittstellen, USB-Schnittstellen, LAN-Schnittstellen, WLAN-Schnittstellen, RFID-Schnittstellen, NFC-Schnittstellen, Bluetooth-Schnittstellen, Infrarot-Schnittstellen beschränkt, können aber so ausgeführt sein.

In bestimmten Ausführungsformen werden Schnittstellen verwendet, die über eine galvanische Entkopplung verfügen. Diese trennen die elektrischen Systeme der am Datenaustausch teilnehmenden Geräte derart, dass kein Strom zwischen diesen ausgetauscht wird. Derartige Schnittstellen können beispielsweise Optokoppler oder Trenntransformatoren umfassen.

Diese Schnittstellen dienen zum Datenaustausch mit einem medizintechnischen Gerät, welches in Figur 1 beispielhaft als Hämodialysegerät 200 ausgeführt ist. Ein Hämodialysegerät ist ein Blutbehandlungsgerät. Andere Blutbehandlungsgeräte, die ebenfalls Daten mit der Schnittstellenvorrichtung 100 austauschen können, sind Geräte zur Peritonealdiaylse, Hämofiltration, Hämodiafiltration oder Plasmapherese oder auch Geräte zur extrakorporalen Herz- und Lungenunterstützung. Grundsätzlich ist jedwedes medizintechnisches Gerät dazu geeignet, Daten mit der Schnittstellenvorrichtung 100 auszutauschen, wenn es über eine entsprechend dazu eingerichtete Schnittstelle verfügt.

Die Software, welche den integrierten Schaltkreis 101 zur Steuerung der Schnittstellenvorrichtung 100 programmiert, kann diesen zur Ausführung vielfältiger Verfahren veranlassen und somit die Schnittstellenvorrichtung 100 einrichten. Diese Verfahren können umfassen:
Prüfen der Daten auf Datensicherheit, die von einer oder mehreren der Schnittstellen 110 bis 114 von einer entfernten Vorrichtung 300, 400 eintreffen. Diese Prüfung umfasst beispielsweise das Abfragen der Herkunft der Daten über die mit den Daten gesendeten IP-Adressen oder Hardwareadressen (MAC-Adressen), das Prüfen von Dateninhalten auf schädliche Inhalte über entsprechend in der Schnittstellenvorrichtung hinterlegte Software wie Software zur Abwehr von Malware, Ransomware oder Computerviren, das Prüfen auf Denial-of-Service Angriffe etc. Generell ist es erfindungsgemäß möglich, durch Aktualisieren der Software der Schnittstellenvorrichtung 100 diese zur Abwehr von allem möglichem bekannten unzulässigem Datenverkehr zu programmieren.

Weiterhin können Daten, die von einer oder mehreren der Schnittstellen 110 bis 114 von einer entfernten Vorrichtung (300, 400) eintreffen, gefiltert werden. Dies kann umfassen, die Daten auf bestimmte Inhalte, wie Patientendaten oder Daten, die das medizintechnische Gerät 200 betreffen aus den Daten zu filtern und zur Weitergabe an das medizintechnische Gerät 200 und/oder zu einer entfernten Vorrichtung 300, 400 vorzubereiten.

Weiterhin können gefilterte oder ungefilterte Daten von mehreren entfernten Vorrichtungen 300, 400 zusammengefasst werden, also in Datenpakete gebündelt werden. Diese Datenpakete können ein bestimmtes, proprietäres Datenformat aufweisen, das von den Vorrichtungen 300,400 oder auch dem medizintechnischen Gerät 200 durch entsprechende Programmierung verarbeitet werden kann.

In einer Ausführungsform ist die Schnittstellenvorrichtung dazu eingerichtet, Daten des medizintechnischen Geräts 200 zu verschlüsseln und/oder verschlüsselte Daten der entfernten Vorrichtungen 300, 400 zu entschlüsseln. Diese Ver- und Entschlüsselungsverfahren sind durch die aktualisierbare Software frei wählbar und aktualisierbar.

In einer bestimmte Ausführungsform ist die Schnittstellenvorrichtung 100 dazu eingerichtet, Daten mit dem medizintechnischen Gerät 200 über eine serielle Schnittstelle, insbesondere eine RS 232 Schnittstelle 113a oder eine USB-Schnittstelle 114a, auszutauschen.

In einer weiteren Ausführungsform umfasst die Schnittstellenvorrichtung eine Datentransformierungseinheit 101a zur Überführung von Daten aus dem Datenaustausch zwischen dem medizintechnischen Gerät 200 und mindestens einer entfernten Vorrichtung 300, 400 von einem ersten in ein zweites Datenformat und/oder umgekehrt. Diese Datentransformierungseinheit 101a ist dazu eingerichtet, die Datenformate der beteiligten Vorrichtungen in das jeweilig andere zu überführen. So arbeiten medizintechnische Geräte, insbesondere solche, die schon älter sind, häufig mit proprietären Datenformaten, die nicht kompatibel zu heutigen gebräuchlichen Datenformaten innerhalb des klinischen Umfelds wie HL7 (Health Level 7) sind. Die Datentransformierungseinheit 110a ist entsprechend dazu programmiert bzw. eingerichtet, die Daten der beteiligten Vorrichtungen in das Format zu überführen, das jeweils für die einzelne Vorrichtung, an die die Daten gesendet werden, kompatibel ist. Die Datentransformierungseinheit 110a kann als Software ausgeführt sein, welche von dem integrierten Schaltkreis 101 ausgeführt wird. Die Datentransformierungseinheit 110a kann auch ein dezidierter integrierter Schaltkreis sein (ASIC), der für diese Aufgabe eingerichtet ist. Entsprechend diesen Ausführungsformen ist die Datentransformierungseinheit 110a in Figur 1 gestrichelt innerhalb des integrierten Schaltkreises dargestellt. Eine Ausführungsform als dezidierter integrierter Schaltkreis, der unabhängig vom dem integrierten Schaltkreis 101 arbeitet, ist ebenfalls möglich.

In bevorzugten Ausführungsformen ist die Schnittstellenvorrichtung 100 dazu eingerichtet, den Datentransfer von dem medizintechnischen Gerät 200 zur Schnittstellenvorrichtung 100 unbeeinflussbar von einer aktualisierten Software, die den integrierten Schaltkreis 101 steuert, zu halten. Dies kann auf Basis der aktualisierten Software selbst realisiert werden, indem diese aktualisierte Software keinen Code enthält, der den entsprechenden Datentransfer von dem medizintechnischen Gerät 200 zur Schnittstellenvorrichtung 100 verändern würde.

Eine weitere Möglichkeit, den Datentransfer von dem medizintechnischen Gerät 200 zur Schnittstellenvorrichtung 100 unbeeinflussbar von einer aktualisierten Software zu machen, besteht darin, die Schnittstellen 110a bis 114a als nicht programmierbare Schnittstellen auszuführen. Diese sind i.d.R. als entsprechende integrierte Schaltkreise ausgeführt in Verbindung mit den mechanisch ausgeführten elektrischen Verbindungen wie Steckleisten oder Buchsen.

In einer weiteren Ausführungsform kann vorgesehen sein, die Schnittstellen 110a bis 114a für eine erste Konfiguration zum Datenaustausch mit einem bestimmten medizintechnischen Gerät 200 programmierbar zu machen und nach erfolgter Konfiguration für eine erneute Programmierung zu sperren. Dies kann beispielsweise dadurch geschehen, in dem elektrische Leiterbahnen, die zur Programmierung der Schnittstellen notwendig sind, mechanisch oder durch Überstrom (sog. Fusing) zerstört werden. Hierdurch wird ein erneutes Programmieren verhindert. Eine Alternative zur Zerstörung der entsprechenden Leiterbahnen besteht darin, die elektrischen Verbindungen wiederherstellbar zu unterbrechen, beispielsweise durch Entfernen von Steckbrücken (engl. "Jumper") von Steckkontakten, wodurch eine elektrische Verbindung unterbrochen wird, im Bedarfsfall aber durch Wiederaufstecken der Steckbrücke wiederherstellbar ist. Hierdurch wird ein erneutes Programmieren erschwert.

In Figur 1 wird diese Unbeeinflussbarkeit des Datentransfers von dem medizintechnischen Gerät 200 zur Schnittstellenvorrichtung 100 durch das Kästchen 103 mit eingezeichnetem Schloss symbolisiert. Dies ist nicht als explizite Vorrichtung zu verstehen, sondern symbolisiert die Trennung der Datenbehandlung durch die Schnittstellenvorrichtung in einen Bereich 104, in dem Daten von dem medizintechnischen Gerät 200 stets in immer derselben Art und Weise unbeeinflussbar empfangen werden, und in einen Bereich 105, in dem ein Datenaustausch mit entfernten Vorrichtungen geschieht, in welchem dieser Datenaustausch von einer aktualisierten Software, die den integrierten Schaltkreis 101 steuert, beeinflussbar ist.

Diese Trennung stellt sicher, dass Daten von dem medizintechnischen Gerät immer in derselben Art und Weise und unbeeinflussbar von der Programmierung der Schnittstellenvorrichtung 100 zu dieser gelangen. Dies ist im medizinischen Umfeld von besonderer Bedeutung, da diese Daten für den Behandlungserfolg und die Sicherheit eines Patienten, der mit der medizintechnischen Vorrichtung behandelt wird, von enormer Bedeutung sind. Es ist deshalb besonders wichtig, dass diese Daten zuverlässig und unbeeinflussbar empfangen werden. Es ist aber auch wichtig, Daten, die von entfernten Vorrichtungen 300,400 empfangen werden, auf Datensicherheit zu überprüfen, diese zu filtern, gegebenenfalls zu entschlüsseln und/oder zu bündeln. Da die entfernten Vorrichtungen 300, 400 vielfältiger Natur sein können und darüber hinaus stetiger technischer Entwicklung unterliegen, genauso wie die Herausforderungen hinsichtlich Datensicherheit sich stets weiterentwickeln, ist es wichtig, die Datenschnittstelle diesbezüglich anpassbar zu gestalten. Dies wird durch die Möglichkeit, die Software, welche den integrierten Schaltkreis 101 steuert, zu aktualisieren, möglich gemacht. Dementsprechend wird durch die Schnittstellenvorrichtung 100 ein gesicherter Datentransfer vom medizintechnischen Gerät 200 zur Schnittstellenvorrichtung 100 und ein sicherer Datenaustausch mit entfernten Vorrichtungen 300, 400 ermöglicht.

Das zugrundeliegende erfindungsgemäße Verfahren zum Steuern der softwaregesteuerten Schnittstellenvorrichtung 100 umfasst, dass die Software wiederholbar in die Schnittstellenvorrichtung 100 eingespeichert werden kann, so dass dieses aktualisierbar ist, und die Schnittstellenvorrichtung 100 zum Datenaustausch zwischen einem medizintechnischen Gerät 200 und mindestens einer entfernten Vorrichtung 300, 400 über mindestens ein Netzwerk 110 bis 114 eingerichtet ist, und ein Aktualisieren der Software einen Datentransfer vom medizintechnischen Gerät 200 zur Schnittstellenvorrichtung 100 nicht beeinflusst.

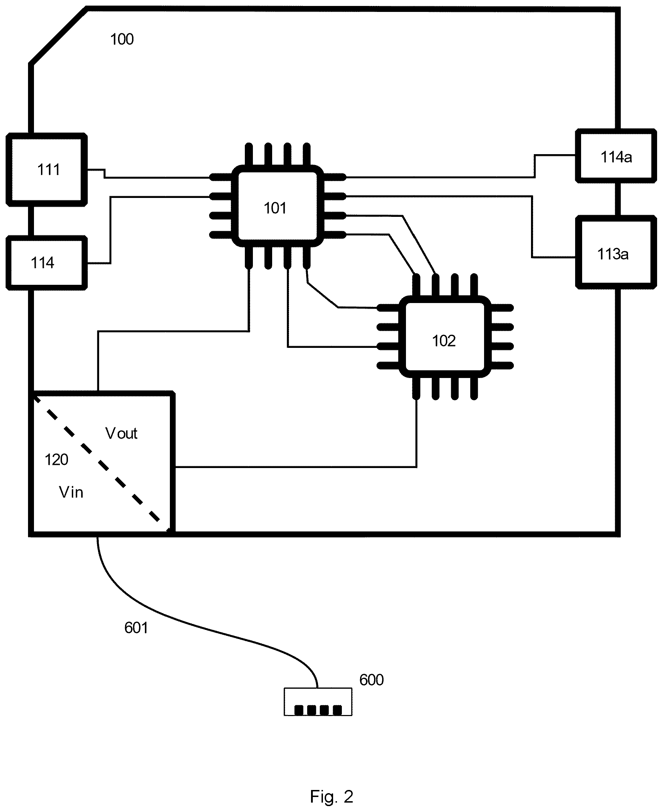

In einer weiteren Ausführungsform erfolgt die Versorgung der Schnittstellenvorrichtung mit elektrischer Energie durch das medizintechnische Gerät 200. Dies ist in Figur 2 schematisch dargestellt.

Die Schnittstellenvorrichtung 100 nach Figur 2 zeigt den integrierten Schaltkreis 101, sowie die Speichervorrichtung 102, die über symbolisierte Leiterbahnen elektrisch miteinander und mit einer Vorrichtung 120 zur Versorgung der Schnittstellenvorrichtung 100 mit elektrischer Energie verbunden sind. Diese Vorrichtung 120 ist das zu eingerichtet, mit einer oder mehreren Spannungsquellen des medizintechnischen Geräts 200 elektrisch verbunden zu werden. Die Vorrichtung 120 ist weiterhin dazu eingerichtet, aus der einen oder mehreren Spannung(en), die somit als Eingangsspannung(en) Vin dienen, eine oder mehrere Ausgangsspannungen Vout zu generieren, die zur Versorgung der Schnittstellenvorrichtung mit elektrischer Energie dienen. Eine derartige Vorrichtung 120 kann ein AC/DC Wandler zur Wandlung von Wechselspannung in Gleichspannung und/oder ein DC/DC Wandler zur Wandlung von Gleichspannung in eine andere Gleichspannung umfassen. Der Fachmann kennt vielfältige, insbesondere auch integrierte Schaltungen, die diese Aufgaben bewältigen.

Die Konnektierung der Vorrichtung 120 mit einer oder mehreren Spannungsquellen des medizintechnischen Geräts 200 kann über eine angepasste elektrische Leitung 601, sowie eine an die Gegebenheiten des medizintechnischen Geräts angepasste Steckverbindung 600 realisiert werden. Ein solche Steckverbindung kann beispielsweise eine Buchsenleiste oder eine Steckerleiste sein, oder jedwede zur Konnektierung mit dem entsprechenden Gegenstück des medizintechnischen Geräts eingerichtete elektromechanische Verbindung.

In einer weiteren Ausführungsform umfasst die Schnittstellenvorrichtung 100 eine Vorrichtung zur Montage der Schnittstellenvorrichtung 100 innerhalb des medizintechnischen Geräts 200. Diese Vorrichtung (nicht in Figur 200 dargestellt) ist an die Gegebenheiten des medizintechnischen Geräts angepasst und kann beispielsweise eine entsprechend ausgeführte Schiene, eine Rastvorrichtung zum Einrasten in entsprechend vorhandene Rastpunkte in dem medizintechnischem Gerät, Schraubaufnahmen, Schraubverbindungen usw. umfassen.

In einer weiteren Ausführungsform ist die Schnittstellenvorrichtung 100 gegen schädliche elektromagnetische Strahlung geschützt. Dies ist insbesondere beim Einbau der Schnittstellenvorrichtung 100 in das medizintechnische Gerät 200 von besonderer Bedeutung, weil Störungen durch elektromagnetische Strahlung die Behandlung des Patienten gefährden können, wenn sie zu Fehlfunktionen des medizintechnischen Geräts führen. Hierzu kann die Schnittstellenvorrichtung über Abschirmbleche oder Abschirmlackierungen verfügen, die Teile der Schnittstellenvorrichtung vor Absendung oder Empfang elektromagnetischer Störstrahlung abschirmen. Ebenfalls ist es möglich, die komplette Schnittstellenvorrichtung 100 innerhalb eines geschirmte Gehäuses zu montieren.

Die Software, welche den integrierten Schaltkreis 101 zur Steuerung der Schnittstellenvorrichtung 100 wie dargelegt programmiert, kann in einem Softwareprodukt vorliegen, das insbesondere auch als maschinenlesbares Medium, wie USB-Stick oder SD-Karte ausgeführt sein kann.

## Patentansprüche

1. Schnittstellenvorrichtung (100) umfassend
zumindest einen von einer Software gesteuerten integrierten Schaltkreis (101),
zumindest eine Speichervorrichtung (102) zur Speicherung der Software,
eine Vielzahl von Schnittstellen (110-114,110a-114a), die zum Datenaustausch zwischen einem medizintechnischen Gerät (200) und mindestens einem entfernten Gerät (300, 400) über mindestens ein Netzwerk eingerichtet sind,
wobei die Schnittstellenvorrichtung (100) so eingerichtet ist, dass der Datenaustausch von dem zumindest einen integrierten Schaltkreis (101) kontrolliert wird,
und dass die Software in die zumindest eine Speichervorrichtung (102) wiederholbar eingespeichert werden kann, so dass diese aktualisierbar ist,
**dadurch gekennzeichnet, dass** die Schnittstellenvorrichtung (100) so eingerichtet ist, dass jeglicher Datentransfer von der medizinischen Vorrichtung (200) zu der Schnittstellenvorrichtung (100) nicht von der aktualisierten Software beeinflussbar ist.

2. Schnittstellenvorrichtung (100) nach Anspruch 1, umfassend eine Datentransformierungseinheit (101a), zur Überführung von Daten aus dem Datenaustausch von einem ersten in ein zweites Datenformat und/oder umgekehrt.

3. Schnittstellenvorrichtung (100) nach einem der vorhergehenden Ansprüche, wobei die Vielzahl von Schnittstellen (110-114,110a-114a) ausgewählt sind aus:
serielle Schnittstellen, parallele Schnittstellen, RS232, USB-Schnittstellen, LAN-Schnittstellen, WLAN-Schnittstellen, RFID-Schnittstellen, NFC-Schnittstellen, 12C-Schnittstellen, Bluetooth-Schnittstellen, Infrarot-Schnittstellen.

4. Schnittstellenvorrichtung (100) nach einem der vorhergehenden Ansprüche, wobei eine serielle Schnittstelle (113a, 114a) zur Datenverbindung mit dem medizintechnischen Gerät 200 eingerichtet ist.

5. Schnittstellenvorrichtung (100) nach einem der vorhergehenden Ansprüche, wobei mehrere Schnittstellen dazu eingerichtet sind, mit mehreren Netzwerken Daten auszutauschen, wobei die Schnittstellenvorrichtung dazu eingerichtet ist, die Daten, die von den mehreren Schnittstellen (110-114,110a-114a) aufgenommen werden, zu filtern und/oder auf Datensicherheit zu überprüfen und/oder in Datenpakete zu bündeln.

6. Schnittstellenvorrichtung (100) nach einem der vorhergehenden Ansprüche, dazu eingerichtet, Daten des medizintechnischen Geräts 200 zu verschlüsseln und/oder verschlüsselte Daten der mindestens einer entfernten Vorrichtung zu entschlüsseln.

7. Schnittstellenvorrichtung (100 nach einem der vorhergehenden Ansprüche, wobei zumindest eine Schnittstelle (110-114,110a-114a) eine galvanisch entkoppelte Schnittstelle ist.

8. Schnittstellenvorrichtung (100) nach einem der vorhergehenden Ansprüche, umfassend eine USB-Schnittstelle (114) oder eine Speicherkartenschnittstelle (115), wobei die Schnittstellenvorrichtung dazu eingerichtet ist, die Software über die USB-Schnittstelle (114) oder über die Speicherkartenschnittstelle (115) zu erhalten.

9. Schnittstellenvorrichtung (100) nach einem der vorhergehenden Ansprüche, umfassend eine Vorrichtung (120) zur Versorgung der Schnittstellenvorrichtung mit elektrischer Energie, wobei die Vorrichtung angepasst ist an eine elektrische Energieversorgung des medizintechnischen Geräts (200).

10. Schnittstellenvorrichtung (100) nach einem der vorhergehenden Ansprüche, umfassend eine Vorrichtung zur Montage der Schnittstellenvorrichtung 100 innerhalb des medizintechnischen Geräts (200), wobei die Vorrichtung angepasst ist an das Innere des medizintechnischen Geräts (200).

11. Medizintechnisches Gerät (200) umfassend eine Schnittstellenvorrichtung 100 nach einem der Ansprüche 1-10.

12. Medizintechnisches Gerät (200) nach Anspruch 11, eingerichtet zur Dialyse.

13. Verfahren zum Steuern einer softwaregesteuerten Schnittstellenvorrichtung (100), wobei die Software wiederholbar in die Schnittstellenvorrichtung (100) eingespeichert werden kann, so dass diese aktualisierbar ist,
**dadurch gekennzeichnet, dass** die Schnittstellenvorrichtung (100) zum Datenaustausch zwischen einem medizintechnischen Gerät (200) und mindestens einer entfernten Vorrichtung (300, 400) über mindestens ein Netzwerk eingerichtet ist und ein Aktualisieren der Software einen Datentransfer vom medizintechnischen Gerät (200) zur Schnittstellenvorrichtung (100) nicht beeinflusst.

14. Softwareprodukt, umfassend Befehle, die bei der Ausführung der Software durch eine Schnittstellenvorrichtung (100) nach einem der Ansprüche 1 bis 10 diese veranlassen, ein Verfahren nach Anspruch 12 auszuführen.

15. Maschinenlesbares Speichermedium, auf dem das Softwareprodukt gemäß Anspruch 14 gespeichert ist.
